# EUROPÄISCHE PATENTANMELDUNG

(11) **EP 1 157 996 A1**
(43) Veröffentlichungstag der Anmeldung: **28.11.2001**
(21) Anmeldenummer: 00110887.7
(22) Anmeldetag: 23.05.2000
(51) Int. Cl.: C07J 41/00, A61K 31/565, A61P 5/36

(54) **Neue Festkörperformen des Mesoprogestins 11Beta-[4E-(Hydroxyiminomethyl)-phenyl]-17Alpha-methoxymethyl-17Beta-methoxy-estra-4,9-dien-3-on**

(71) Anmelder: JENAPHARM GmbH, D-07745 Jena (DE)
(72) Erfinder: Grawe, Detlef, 99510 Kleinromstedt (DE); Hösel, Peter, Dr., 07745 Jena (DE); Müller, Uwe,, 07747 Jena (DE); Winter, Gabriele, Dr., 16567 Schönfliess (DE)
(74) Vertreter: Leybach, Holger Hugo

(57) **Zusammenfassung**

Die vorliegende Erfindung betrifft neue Festkörperformen des Mesoprogestins 11β-[4E-(Hydroxyiminomethyl)phenyl]-17α-methoxymethyl-17β-methoxy-estra-4,9-dien-3-on (Oxim J 867), insbesondere eine hochreine und stabile amorphe bzw. hochkristalline Form (Ansolvat/Anhydrat) der Verbindung J 867, Verfahren zu deren Herstellung sowie deren Verwendung in pharmazeutischen Zusammensetzungen.

Die neuen Festkörperformen zeichnen sich insbesondere durch eine hohe Stabilität aus. Die Festkörperformen des Oxims J 867 können insbesondere in der Fertilitätskontrolle und der Hormon-Ersatz-Therapie verwendet werden.

## Beschreibung

Die vorliegende Erfindung betrifft neue Festkörperformen des Mesoprogestins 11β-[4E-(Hydroxyiminomethyl)phenyl]-17α-methoxymethyl-17β-methoxy-estra-4,9-dien-3-on (Oxim J 867), insbesondere eine hochreine und stabile amorphe bzw. hochkristalline Form (Ansolvat/Anhydrat) der Verbindung J 867, Verfahren zu deren Herstellung sowie deren Verwendung in pharmazeutischen Zusammensetzungen.

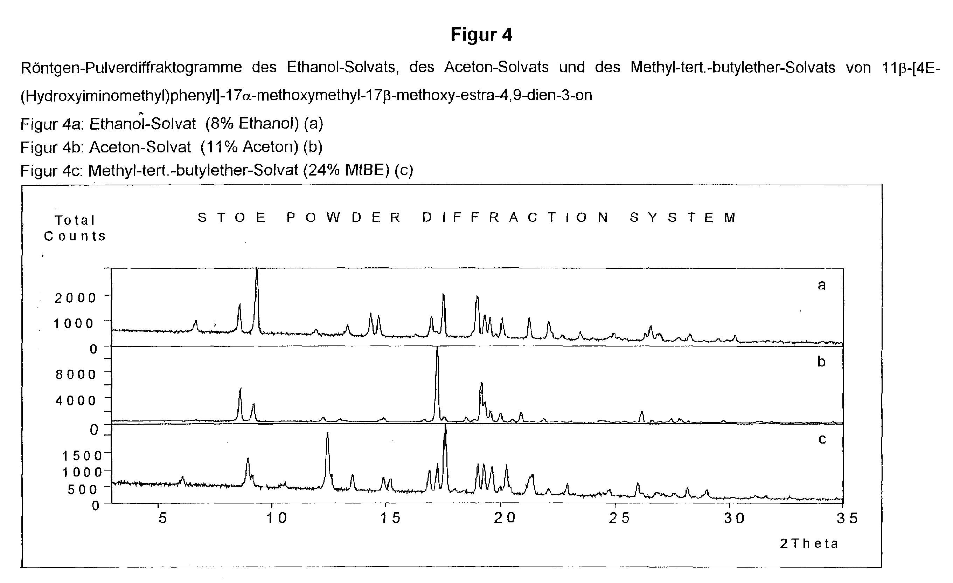

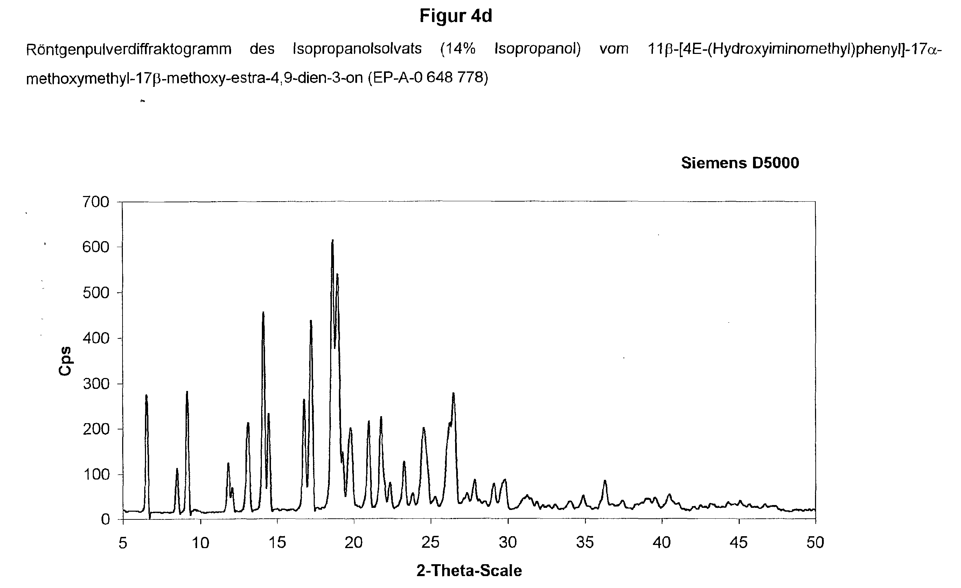

11β-[4E-(Hydroxyiminomethyl)phenyl]-17α-methoxymethyl-17β-methoxy-estra-4,9-dien-3-on ist beispielsweise aus EP-A-0 648 778 bzw. DE-A-43 32 283 bekannt. Die dort nach Umkristallisation aus Methylenchlorid/Isopropanol erhaltene Form schmilzt bei 113 °C. Bei der beschriebenen Kristallform handelt es sich um ein Isopropanol-Solvat mit 14% Isopropanol (siehe XRPD: Tabelle 2/Figur 4d). Kennzeichnend für das Oxim ist seine starke Tendenz aus Lösungsmitteln als Solvat auszukristallisieren. Die Solvatform aber ist für pharmazeutische Anwendungen wenig geeignet. Die Lösungsmittelbindung an die Substanz ist sehr fest und wird erst bei höheren Temperaturen gelöst. Die Oxim-Solvate spalten im allgemeinen erst oberhalb 100°C das Lösemittel vollständig ab. Dabei entstehen je nach Heizrate inhomogene amorph-kristalline Mischformen.

Die Reinigung des Oxims von Nebenprodukten der Oximierungsreaktion (Dioxime, Z-Oxim) erfolgt durch aufwendige Säulenchromatographie mit Toluol/Acetongradienten. Eine Reinigung durch Kristallisationsverfahren wird durch die allgemein schlechten Löslichkeiten in den dafür in Frage kommenden Lösungsmitteln erschwert. Einschränkend kommt hinzu, daß bei höheren Temperaturen in Lösung eine Isomerisierung des E-lsomeren der Verbindung zum Z-lsomeren auftritt.

Aufgabe der Erfindung ist daher durch ein geeignetes Verfahren die aufwendige Säulenchromatographie zu ersetzen und eine homogene, lösungsmittelfreie und stabile feste Form mit guten pharmazeutischen Eigenschaften bereitzustellen.

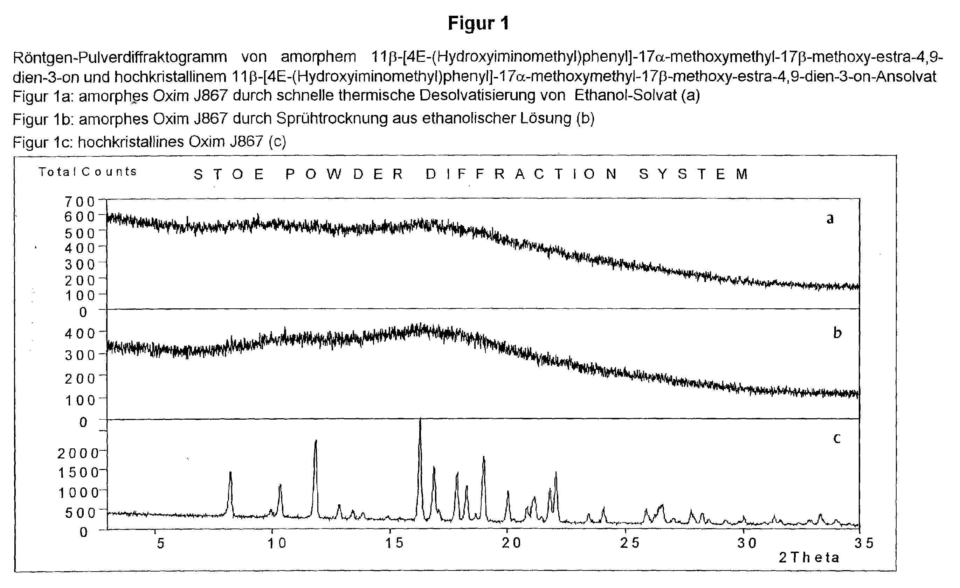

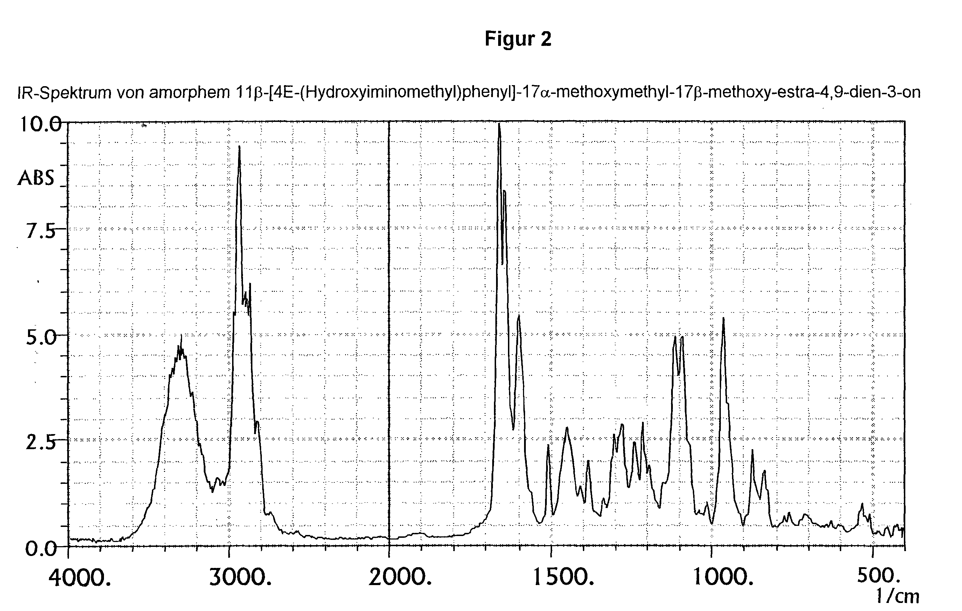

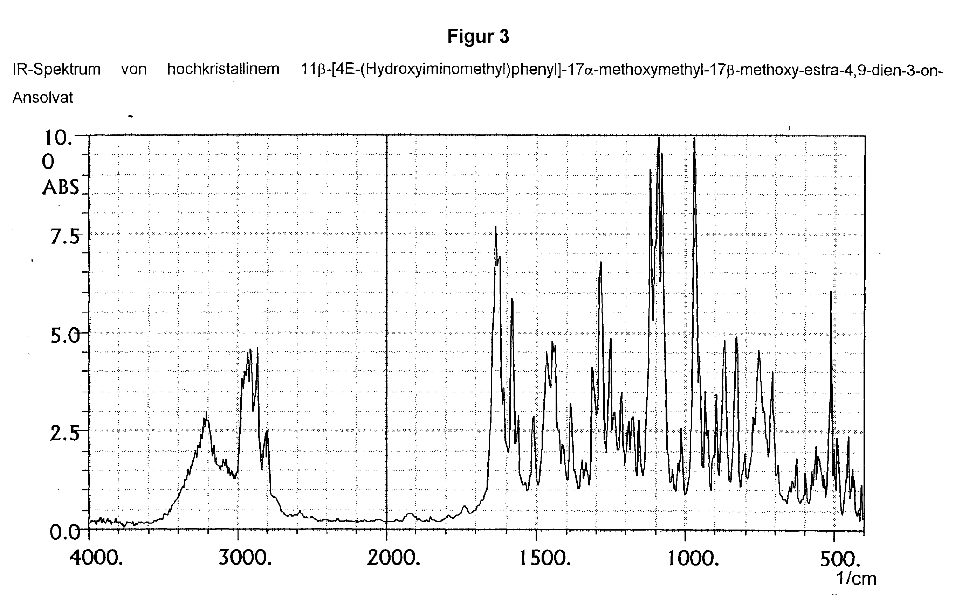

Diese Aufgabe wird gelöst durch amorphes 11β-[4E-(Hydroxyiminomethyl)phenyl]-17α-methoxymethyl-17β-methoxy-estra-4,9-dien-3-on, das dadurch gekennzeichnet ist, daß keine kristallinen Reflexe im Röntgen-Pulverdiffraktögramm (XRPD; Figur 1a/b) zu sehen sind, bzw. dessen Modifikation durch das in Figur 2 dargestellte IR-Spektrum gekennzeichnet ist.
Des weiteren wird diese Aufgabe gelöst durch hochkristallines 11β-[4E (Hydroxyiminomethyl)phenyl]-17α-methoxymethyl-17β-methoxy-estra-4,9-dien-3-on-Ansolvat. Die Kristallform des Ansolvats ist durch das in Figur 1c dargestellte Röntgen-Pulverdiffraktogramm bzw. durch das in Figur 3 dargestellte IR-Spektrum charakterisiert.

Kristalline Solvate von 11β-[4E-(Hydroxyiminomethyl)phenyl]-17α-methoxymethyl-17β-methoxy-estra-4,9-dien-3-on können mittels einem Verfahren hergestellt werden, umfassend die folgenden Schritte:
a) Herstellung eines Solvats von 11β-[4E-(Hydroxyiminomethyl)phenyl]-17α-methoxymethyl-17β-methoxy-estra-4,9-dien-3-on und Auflösen des Solvats oberhalb der Sättigungslöslichkeit in einem Lösungsmittel, insbesondere Toluol, das unterschiedlich vom Solvat-Lösungsmittel ist und in welchem die Solvat-Kristallstruktur instabil ist,
b) gegebenenfalls Zugabe eines weiteren Lösungsmittels als Rekristallisations-Inhibitor, insbesondere Methanol oder Essigester, und
c) Zugabe eines Solvatbildner-Lösungsmittels, insbesondere Ethanol, Aceton und Methyl-tert.-butylether (MtBE) und Kristallisieren des Solvats.

Die kristallinen Solvate von 11β-[4E-(Hydroxyiminomethyl)phenyl]-17α-methoxymethyl-17β-methoxy-estra-4,9-dien-3-on, nämlich das Methyl-tert.-butylether-Solvat, das Aceton-Solvat und das Ethanol-Solvat, wobei die hochkristallinen Formen durch die in Figur 4a/b/c und Tabelle 2 dargestellten Röntgen-Pulverdiffraktogramme gekennzeichnet sind, sind neu und stellen Zwischenstufen auf dem Weg zum amorphen 11β-[4E-(Hydroxyiminomethyl)phenyl]-17α-methoxymethyl-17β-methoxy-estra-4, 9-dien-3-on bzw. zum hochkristallinen 11β-[4E-(Hydroxyiminomethyl)phenyl]-17α-methoxymethyl-17β-methoxy-estra-4,9-dien-3-on-Ansolvat dar.

Durch Trocknen der vorstehend erhaltenen, kristallinen Solvate von 11β-[4E-(Hydroxyiminomethyl)phenyl]-17α-methoxymethyl-17β-methoxy-estra-4,9-dien-3-on, insbesondere durch thermische Desolvatisierung vorzugsweise unter Vakuum und mit einer Heizrate im Produkt von mindestens 0,5°C/min oder aus einer Lösung des Solvats durch Sprühtrocknung (XRPD; Figur 1) unterhalb des Glasübergangspunktes der amorphen Struktur wird dann die amorphe Form von 11β-[4E-(Hydroxyiminomethyl)phenyl]-17α-methoxymethyl-17β-methoxy-estra-4,9-dien-3-on ge-wonnen. Die hochkristalline Form des 11β-[4E-(Hydroxyiminomethyl)phenyl]-17α-methoxymethyl-17β-methoxy-estra-4,9-dien-3-ons kann durch Erwärmen der Solvat- bzw. der amorphen Festkörperform, gegebenenfalls in wässriger Suspension, erzeugt werden. Gegenstand der vorliegenden Erfindung sind auch pharmazeutische Zusammensetzungen, insbesondere feste pharmazeutische Zusammensetzungen, umfassend das vorstehend beschriebene amorphe und/oder hochkristalline 11β-[4E-(Hydroxyiminomethyl)phenyl]-17α-methoxymethyl-17β-methoxy-estra-4,9-dien-3-on, in Kombination mit einem pharmazeutisch verträglichen Träger und/oder Verdünnungsmittel.

Die pharmazeutischen Zusammensetzungen der vorliegenden Erfindung werden mit den üblichen festen oder flüssigen Trägerstoffen oder Verdünnungsmitteln und den üblicherweise verwendeten pharmazeutisch technischen Hilfsstoffen entsprechend der gewünschten Applikationsart mit einer geeigneten Dosierung in an sich bekannter Weise hergestellt.

Unter dem Begriff "hochkristallin" wird erfindungsgemäß ein Zustand verstanden, aus dem heraus keine weitere meßbare Zunahme der Kristallinität (XRPD, DSC) durch Bedingungen festgestellt werden kann, welche die Rekristallisation der amorphen Struktur begünstigen, wie z.B. langanhaltendes Kochen der suspendierten Substanz in Wasser. Der hochkristalline Zustand ist dadurch charakterisiert, daß im DSC bei langsamer Heizrate (1K/min) kein exothermer Rekristallisationspeak zwischen 110°C und 160°C detektiert werden kann. Es existiert ausschließlich der endotherme Schmelzpeak bei 194,7°C ± 2K (Heizrate 5K/min).

In den Figuren 1a bis c sind die Röntgen-Pulverdiffraktogramme von amorphem 11β-[4E-(Hydroxyiminomethyl)phenyl]-17α-methoxymethyl-17β-meth-oxy-estra-4,9-dien-3-on bzw. hochkristallinem 11 β-[4E-(Hydroxyiminomethyl)phenyl]-17α-methoxymethyl-17β-meth-oxy-estra-4,9-dien-3-on-Ansolvat (vgl. Tabelle 1) dargestellt.
In den Figuren 2 und 3 sind IR-Spektren von amorphem 11β-[4E-(Hydroxyiminomethyl)phenyl]-17α-methoxymethyl-17β-methoxy-estra-4,9-dien-3-on bzw. hochkristallinem 11 β-[4E-(Hydroxyiminomethyl)phenyl]-17α-methoxymethyl-17β-methoxy-estra-4,9-dien-3-on-Ansolvat dargestellt.
In Figur 4 (Tabelle 2) sind Röntgen-Pulverdiffraktogramme des Methyl-tert.-butylether-Solvats, des Aceton-Solvats, des Ethanol-Solvats und des Isopropanol-Solvats (vgl. Tabelle 2) von 11β-[4E-(Hydroxyimino-methyl)phenyl]-17α-methoxymethyl-17β-methoxy-estra-4,9-dien-3-on dargestellt.
In Figur 5 ist ein Röntgen-Pulverdiffraktogramm von amorphem 11β-[4E-(Hydroxyiminomethyl)phenyl]-17α-methoxymethyl-17β-methoxy-estra-4,9-dien-3-on zum Zeitpunkt 0 (Start), nach 12 Monaten bei 25 °C und nach 12 Monaten bei 40 °C dargestellt.
In Figur 6 ist die Löslichkeit von hochkristallinem 11β-[4E-(Hydroxyiminomethyl)phenyl]-17α-methoxymethyl-17β-methoxy-estra-4,9-dien-3-on-Ansolvat bzw. amorphem 11β-[4E-(Hydroxyiminomethyl)phenyl]-17α-methoxymethyl-17β-methoxy-estra-4,9-dien-3-on in Wasser mit und ohne Tensid gezeigt.

**Tabelle 1:**

| XRPD-Daten des hochkristallinen Oxims J867 - d-Werte(d_{obs}), relative Intensitäten (l_{obs}) and hkl-Werte der stärksten Peaks | | | | | |
|---|---|---|---|---|---|
| **d**_{**obs**} **[Å]** | **l**_{**obs**} | **h k l** | **d**_{**obs**} **[Å]** | **l**_{**obs**} | **h k l** |
| 10,73 | 40,0 | 1 1 0 | 4,15 | 6,1 | 022 |
| 8,88 | 7,6 | 101 | 4,08 | 30,5 | 1 3 1* 410 |
| 8,53 | 33,9 | 2 0 0 | 4,03 | 47,0 | 1 2 2 |
| 7,46 | 80,5 | 1 1 1 | 3,79 | 8,8 | 411 |
| 6,88 | 11,7 | 0 2 0 | 3,70 | 13,0 | 312 |
| 6,59 | 8,1 | 2 0 1 | 3,44 | 12,2 | 0 3 2 |
| 6,38 | 6,8 | 1 2 0 | 3,36 | 18,8 | 013 |
| 5,95 | 3,4 | 2 1 1 | 3,30 | 5,9 | 4 0 2 |
| 5,44 | 100,0 | 121 | 3,21 | 10,9 | 412 |
| 5,26 | 55,2 | 3 1 0 | 3,16 | 11,8 | 5 1 1 |
| 5,20 | 11,3 | 0 0 2 | 3,13 | 4,7 | 4 3 0 |
| 4,97 | 47,1 | 102 | 3,05 | 4,6 | 2 4 1 |
| 4,86 | 36,5 | 0 1 2 | 2,97 | 5,8 | 4 2 2 |
| 4,76 | 5,9 | 221 | 2,89 | 2,9 | 313 |
| 4,68 | 65,9 | 1 1 2 | 2,85 | 7,6 | 502 |
| 4,44 | 27,4 | 202* 1 3 0 | 2,83 | 4,1 | 1 4 2 |
| 4,27 | 12,9 | 400 | 2,69 | 9,7 | 403* 6 1 1 |
| 4,20 | 23,6 | 0 3 1 | 2,64 | 4,6 | |

| | | | | | |
|---|---|---|---|---|---|
| * Beide hkl-Werte sind möglich, da die Peak-Trennung begrenzt ist auf 2Θ = 0,08° (angulare Auflösung des Detektors). | | | | | |

**Tabelle 2:**

| XRPD-Daten der Solvate des Oxims J867 - d-Werte (d_{obs}) and relative Intensitäten (l_{obs}) der stärksten Peaks | | | | | | | |
|---|---|---|---|---|---|---|---|
| **Ethanol-Solvat** | | **Methyl tert.-butyl ether- Solvat** | | **Aceton- Solvat** | | **Isopropanol- Solvat** | |
| **d**_{**obs**} **[Å]** | **l**_{**obs**} | **d**_{**obs**} **[Å]** | **l**_{**obs**} | **d**_{**obs**} **[Å]** | **l**_{**obs**} | **d**_{**obs**} **[Å]** | **l**_{**obs**} |
| 13,22 | 19,3 | 14,47 | 13,7 | 10,24 | 42,1 | 13,42 | 52,1 |
| 10,27 | 44,4 | 9,86 | 43,9 | 9,58 | 22,8 | 10,34 | 17,8 |
| 9,44 | 100,0 | 9,67 | 18,3 | 7,21 | 5,9 | 9,61 | 51,3 |
| 7,39 | 8,9 | 8,36 | 6,6 | 6,81 | 3,3 | 7,48 | 21,4 |
| 6,64 | 16,6 | 7,10 | 81,7 | 6,68 | 2,0 | 7,33 | 15,6 |
| 6,17 | 39,6 | 6,53 | 22,6 | 5,99 | 3,2 | 6,76 | 36,4 |
| 6,03 | 34,4 | 5,94 | 20,5 | 5,94 | 5,0 | 6,28 | 80,2 |
| 5,21 | 35,6 | 5,82 | 19,5 | 5,32 | 3,2 | 6,13 | 45 |
| 5,06 | 68,7 | 5,24 | 32,3 | 5,14 | 100,0 | 5,29 | 51,6 |
| 4,67 | 72,3 | 5,14 | 42,5 | 5,05 | 6,8 | 5,14 | 69,1 |
| 4,59 | 40,8 | 5,04 | 100,0 | 4,79 | 5,8 | 4,77 | 100,0 |
| 4,54 | 30,3 | 4,92 | 5,6 | 4,70 | 3,3 | 4,69 | 94,4 |
| 4,48 | 6,3 | 4,67 | 42,9 | 4,63 | 51,7 | 4,60 | 22,1 |
| 4,42 | 30,0 | 4,60 | 42,6 | 4,59 | 26,0 | 4,49 | 31,5 |
| 4,17 | 33,5 | 4,52 | 38,5 | 4,53 | 14,3 | 4,23 | 39,7 |
| 4,02 | 25,8 | 4,44 | 9,7 | 4,44 | 11,3 | 4,08 | 38,3 |
| 3,91 | 6,9 | 4,38 | 1,7 | 4,32 | 3,6 | 3,97 | 14,9 |
| 3,78 | 12,5 | 4,16 | 28,8 | 4,25 | 12,6 | 3,82 | 23,8 |
| 3,70 | 4,5 | 4,02 | 9,2 | 4,06 | 4,7 | 3,73 | 11,6 |
| 3,59 | 7,8 | 3,88 | 15,9 | 3,65 | 2,7 | 3,62 | 37,1 |
| 3,57 | 13,3 | 3,66 | 5,0 | 3,63 | 2,1 | 3,59 | 22,4 |
| 3,54 | 7,0 | 3,63 | 5,4 | 3,40 | 14,6 | 3,41 | 35,0 |
| 3,50 | 4,2 | 3,59 | 9,6 | 3,34 | 3,5 | 3,39 | 35,9 |
| 3,38 | 10,0 | 3,42 | 20,5 | 3,30 | 2,1 | 3,36 | 37,61 |
| 3,35 | 23,9 | 3,32 | 7,8 | 3,25 | 4,8 | 3,26 | 11,8 |
| 3,32 | 12,5 | 3,29 | 5,5 | 3,20 | 5,0 | 3,2 | 14,0 |
| 3,30 | 12,7 | 3,23 | 6,4 | 3,16 | 2,0 | 3,07 | 16,4 |
| 3,21 | 6,3 | 3,17 | 14,5 | 3,00 | 3,4 | 3 | 13,6 |
| 3,15 | 10,7 | 3,07 | 12,2 | 2,86 | 2,1 | 2,47 | 12,65 |
| 3,02 | 4,9 | 2,87 | 5,6 | 2,85 | 2,2 | 2,23 | 10,6 |
| 2,98 | 3,7 | 2,83 | 4,6 | 2,81 | 2,2 | | |
| 2,95 | 10,6 | | | 2,59 | 2,4 | | |

Die Erfindung betrifft eine neue hochreine und stabile amorphe bzw. hochkristalline Form des Mesoprogestins 11β-[4E-(Hydroxyiminomethyl)phenyl]-17α-methoxymethyl-17β-methoxy-estra-4,9-dien-3-on (J 867): 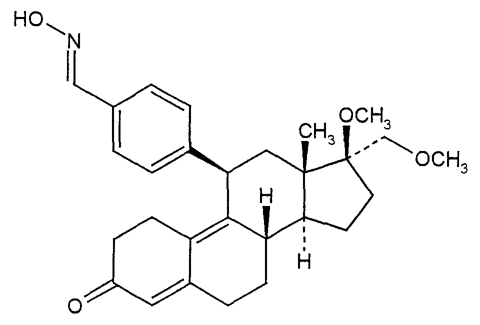 und Solvat-Formen von J867, die Zwischenprodukte zur Herstellung der amorphen bzw. der hochkristallinen Form darstellen. Als Problem bei der Herstellung der amorphen bzw. hochkristallinen Form hat sich erwiesen, daß die Substanz im Stadium der Desolvatisierung ein amorphes Zwischenstadium durchläuft, aus dem je nach Heizrate, Endtemperatur und Trocknungsdauer mehr oder weniger vollständig die Ansolvat/Anhydrat-Form rekristallisiert. Der Grad der Kristallinität ist jedoch schwer zu steuern, so daß inhomogene Mischformen aus amorphen und kristallinen Anteilen entstehen. Außerdem führt die thermische Belastung der zur Z-lsomerisierung und Oxim-Spaltung neigenden chemischen Struktur zu einer Verschlechterung der Reinheit.

Die Herstellung der für die feste Endform des Oxims geeigneten Solvatform kann erfindungsgemäß mit der Abtrennung des Z-lsomeren und der Dioxime verbunden werden. Durch eine gezielte Ausnutzung der thermodynamischen Instabilität geeigneter Solvate, vorzugsweise des Solvats des Methyl-tert.-butylethers (MTBE), in einer dafür geeigneten Lösemittelumgebung, ist eine vollständige Auflösung des Solvatgitters weit oberhalb der Löslichkeitsgrenze und schon in der Nähe der Raumtemperatur möglich. Im Kontakt mit Toluen im Massenverhältnis von 1:2 bis 1:1 löst sich die Kristallform des MTBE-Solvats, trotz der nur geringen Löslichkeit in Toluol von nur 3,3 Gew%, schon bei 20-35°C schnell und vollständig auf. Es erfolgt ein sogenanntes kaltes Schmelzen. Dieser Zustand kann durch Zugabe geeigneter Lösungsmittel als Rekristallisations-Inhibitoren für technische Anwendungen stabilgehalten werden. So kann der Übersättigungszustand in toluenischer Lösung durch Zugabe von nur 5 Vol.-% Methanol für Stunden stabilisiert werden. Anschließend wird durch Zugabe des Solvatbildner-Lösungsmittels (MTBE) in geeigneter Konzentration (z.B. 100 - 130 Vol.-% MTBE, bezogen auf eingesetztes Toluen) das Solvatgitter wieder aufgebaut, wobei die Verunreinigungen größtenteils in Lösung bleiben.

Durch ein besonders schnelles Trocknungsverfahren, vorzugsweise Sprühtrocknung, unterhalb des Glasübergangspunktes direkt aus der Lösung des Oxim-Solvates J867 in einem organischen Lösungsmittel, vorzugsweise Ethanol, kann eine stabile, vollständig amorphe, lösungsmittelfreie Struktur gewonnen werden. Hierzu wird in Ethanol zu 3 bis 13 Gew.-% gelöstes Oxim über ein geeignetes Zerstäubungsaggregat (Zweistoffdüse oder Zentrifugalzerstäuber) in heißem Stickstoff versprüht und in extrem kurzer Zeit zu amorphen, mikrofeinen Partikeln getrocknet. Die Eintrittstemperatur des Stickstoffs beträgt dabei 120°C bis 200°C, vorzugsweise 150°C-180°C. Das Verhältnis Oximlösung zu Trocknungsgas sollte dabei 0,01 bis 0,3 kg Lösung /m³ N₂, vorzugsweise 0,08 bis 0,12 kg Lösung /m³ N₂, betragen. Der Trocknungsprozeß wird so gesteuert, daß im Trocknerausgang eine Temperatur von 55°C bis 95°C, vorzugsweise 75 bis 85°C, eingestellt wird. Diese Temperatur liegt deutlich unter der Glasübergangstemperatur des amorphem Oxims (105°C), wo die Substanz eine gel/glasartige Konsistenz annimmt und oberhalb derer eine mehr oder weniger schnelle bzw. spontane Rekristallisation stattfinden kann.

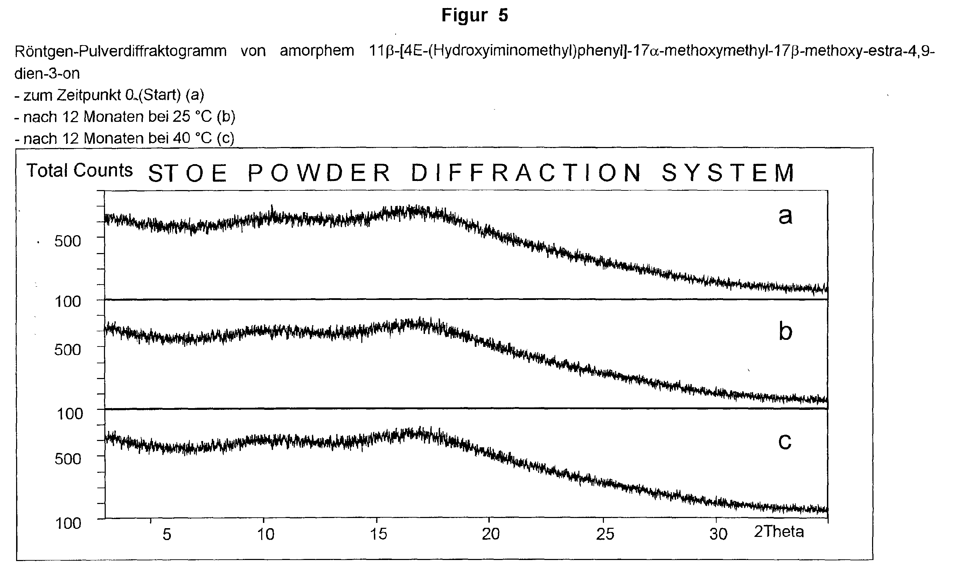

Durch Anwendung der Sprühtrocknung wird eine lösungsmittelfreie, homogene und mikrofeine Festkörperform von Oxim J 867 in einem Verfahrensschritt erhalten.
Der Vorteil der Verwendung der erfindungsgemäßen amorphen Form von Oxim J867 zur Herstellung von Arzneimitteln liegt darin, daß diese amorphe Struktur neben den guten Löslichkeitseigenschaften überraschenderweise eine sehr gute Lagerstabilität aufweist. Auch nach 12 Monaten im accelerated ICH-Stability-Test (40°C, 75% relative Feuchtigkeit) zeigt die amorphe Struktur keine Anzeichen einer Rekristallisation und/oder chemischen Zersetzung (siehe Figur 5). Auch während der pharmazeutischen Verarbeitung (Feuchtgranulierung, Tablettierung) bleibt die physikalische Struktur des Oxims stabil. Das heißt, eine Umwandlung der amorphen in die hochkristalline Form bzw. eine E/Z-Isomerisierung tritt nicht auf. Durch die Stabilität werden negative Einflüsse derartiger Umwandlungen auf die Bioverfügbarkeit des Wirkstoffes vermieden. Bezüglich der Lagerstabilität gilt sinngemäß das gleiche für die Verwendung der erfindungsgemäßen hochkristallinen Form des Oxims J 867, wobei erwartungsgemäß die chemische Stabilität besonders unter Streß (hohe Temperaturen und Feuchtigkeiten) höher als die der amorphen Form ist.

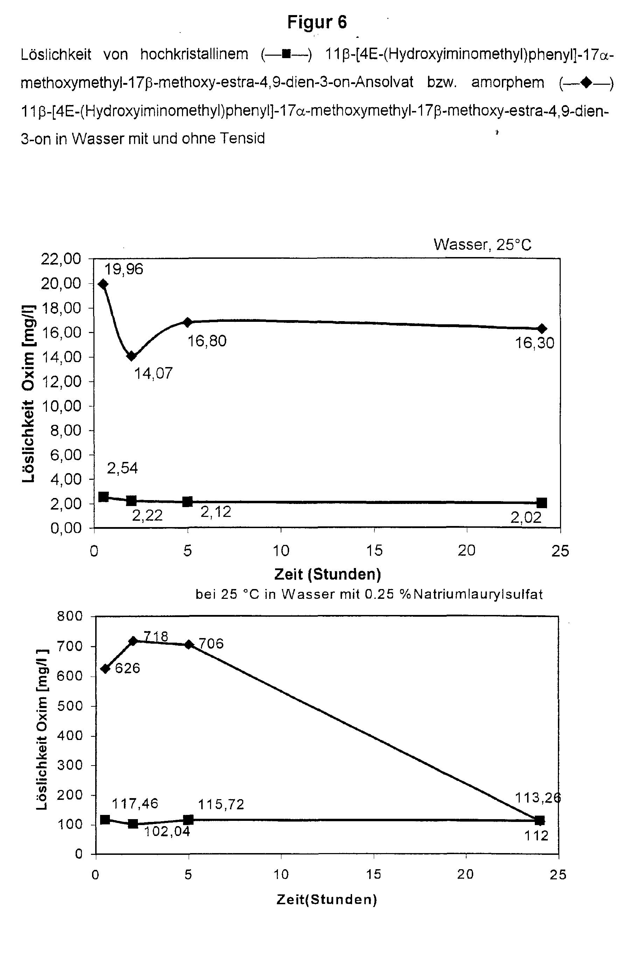

Des weiteren besitzt die amorphe Form hervorragende Löslichkeitseigenschaften. So ist die Sättigungslöslichkeit in Wasser als auch in Tensidlösung (0,25 %-iges Natriumlaurylsulfat) etwa 7 bis 8 mal höher als die der hochkristallinen Form, wobei dieses hohe Übersättigungsninevau für mehrere Stunden, wie im Falle von Wasser für über 24 h stabil bleibt (siehe Figur 6).

Die Herstellung der hochkristallinen Form kann ausgehend von einer der Solvatformen des Oxims J867 erfolgen. Die Solvatform kann hierzu in Wasser bei einer Temperatur von 50 bis 100 °C suspendiert werden, dabei spaltet sich schon weit unterhalb der Desolvatisierungstemperatur der trockenen Solvatform das Lösungsmittel ab. Im Fall des MTBE-Solvates führt bereits eine nur kurzzeitige Behandlung (10 bis 30 min) mit Wasser bei einer Temperatur von 65 bis 75°C zur vollständigen Umwandlung in die hochkristalline Form.

Mit dem erfindungsgemäßen Verfahren, insbesondere der thermischen Desolvatisierung ist es grundsätzlich möglich, je nach Prozeßbedingungen amorph-kristalline Mischformen zu erzeugen, die jedoch nicht reproduzierbar erhältlich und für die Entwicklung eines validierten Herstellungsverfahrens für die Qualitätskontrolle, für die pharmazeutische Weiterverarbeitung und für die Zulassung erhebliche Probleme darstellen können.

Das Oxim J867 ist gemäß EP-A-0 648 778 bzw. DE-A-43 32 283 eine antigestagen wirkende Substanz, die bei gleicher Aktivität wie RU 486 (Mifepriston) am Progesteron-Rezeptor eine im Vergleich mit RU 8486 deutlich reduzierte antiglucocortikoide Aktivität besitzt.

In den US-Anmeldungen Nr. 09/386,141, 09/386,140 und 09/386,133 wird das Oxim J 867 als Mesoprogestin bezeichnet. Mesoprogestine sind hierbei als Verbindungen definiert, die in vivo sowohl agonistische als auch antagonistische Aktivität am Progesteron-Receptor (PR) aufweisen. Entsprechende funktionale Zustände können mit Gestagen und Antigestagen nicht erreicht werden. Das Oxim J 867 eignet sich insbesondere für folgende Verwendungen:
a) Das Oxim J 867 kann gemäß US-Anmeldung Nr. 09/386,133, gegebenenfalls zusammen mit einem Estrogen, zur Herstellung eines Arzneimittels zur weiblichen Kontrazeption verwendet werden.
b) Das Oxim J867 kann gemäß US-Anmeldung Nr. 09/386,141 zur Behandlung und Verhinderung von benignen hormonabhängigen gynäkologischen Störungen; wie
   - zur Behandlung von gynäkologischen Störungen, wie Endometriose, uterinen Fibroiden, postoperativen peritonealen Adhäsionen, dysfunktionaler Blutung (Metrorrhagie, Menorrhagie) and Dysmenorrhöe;
   - zur Verhinderung von gynäkologischen Störungen; wie postoperativen, peritonealen Adhäsionen, dysfunktionaler Uterusblutung (Metrorrhagie, Menorrhagie) und Dysmenorrhöe, verwendet werden. Die tägliche Dosis des Mesoprogestins beträgt 0,5 bis 100 mg, vorzugsweise 5,0 bis 50 mg und am stärksten bevorzugt 10 bis 25 mg.
c) Das Oxim J867 kann gemäß US-Anmeldung Nr. 09/386,140 ebenso als pharmazeutischer Bestandteil zur Herstellung eines Arzneimittels gegebenenfalls zusammen mit einem Estrogen in der Hormon-Replacement-Therapy (HRT) und zur Behandlung von Hormonmangel und Symptomen von Hormon-Irregularität eingesetzt werden.

### Meßverfahren

### Röntgenpulverdiffraktometrie (X-Ray Powder Diffraction; XRPD):

Die Daten wurden mit einem STOE Powder Diffraktometer STADIP mit Germaniummonochromatischer CuKα₁-Strahlung (λ = 1.540598 Å) - zwischen 3° ≤ 2Θ ≤ 35° bzw. einem Siemens Diffraktometer D5000 mit Cu-Anode ( 5° ≤ 2Θ ≤ 50°) ermittelt.

### IR-Spektroskopie:

Verwendet wurde ein NICOLET 20 SXB mit Photoacoustic Detector MTEC (KBr, 8t, 90 Sekunden).

### Löslichkeitsuntersuchungen:

200 mg Substanz wurden bei 25°C in 50 ml Wasser (ohne und mit 0,25% SDS (Natriumlaurylsulfat)) gegeben. Proben wurden nach 0,5 h, 2 h, 5 h und 24 h genommen.
Probenahme: 0,45 µm Filtervorsatz, Verdünnung 1:1 mit MeOH, HPLO.

### HPLC:

Die Reinheitsbestimmung erfolgt nach folgender Methode:
Säule: Superspher Si 60, 250x4 mm, 4 µm
Eluent: Chloroform (3% Ethanol)/Wasser wie 96,9/0,1
Fluß: 1 ml/min
Detektion: UV (299 nm)
Auswertung: 100%-Flächennormalisierung

### Headspace für Restlösungsmittel:

GC-Autosystem mit HS40 Perkin Elmer, Säule DB-Wax, 30 m x 0,23 mm, FID.

### Korngrößenverteilung:

Sympatec HELOS (H0445), Trockendispergiersystem(RODOS), Druck 2 bar.

### DSC:

Perkin Elmer DSC 7 und NETZSCH DSC 200/1/F in Pan-AI, pierced lid, Stickstoff.

Die Erfindung wird durch die nun folgenden Beispiele näher erläutert.

### Beispiele

### Beispiel 1

2 kg Oxim J867 (MtBE-Solvat mit 20 bis 24 % MtBE; soweit nicht anders angegeben, sind die in den Solvaten enthaltenen Prozent an Lösungsmittel Gewichtsprozent) werden in 20 l Ethanol bei 50°C unter Rühren gelöst. Die Lösung wird in einem unter inerten Bedingungen (Stickstoff) betriebenen Sprühtrockner im Gleichstromverfahren sprühgetrocknet. Die Trockenkammer des Sprühtrockners wird mit 60 m³/h Stickstoff, der auf 175°C vorgeheizt ist, beschickt. Anschließend wird über eine Schlauchpumpe die Lösung mit 6,4 l/h über eine Zweistoffdüse, die mit 3 bar Stickstoff als Treibgas betrieben wird, in der Trockenkammer versprüht. Die Trocknungsgasaustrittstemperatur beträgt 79°C - 83°C. Das zu mikrofeinen Partikeln getrocknete Oxim J867 wird in einem Produktfilter vollständig aufgefangen.
Das Oxim J867 weist folgende Qualitätsparameter auf:

| | |
|---|---|
| Restlösemittel | 0,44% Ethanol, 0,11% MtBE |
| Wasser | 0,5% |
| Korngröße | 100% < 12,5 µm |
| | 50% < 2,25 µm |
| | 10% < 0.82 µm |
| Reinheit (Gehalt an Z-Oxim) | keine Z-lsomerisierung während der thermischen Belastung: |
| | 1,7 F% (Flächenprozent) vor Trocknung |
| | 1,7 F% nach Trocknung |
| XRPD | 100% amorph - keine kristallinen Reflexe |

### Beispiel 2

Zu 20 g Oxim J867 (MTBE-Solvat mit 20% - 24% MtBE) werden 50 ml Toluen gegeben. Die Substanz löst sich bei 25°C sehr schnell und vollständig auf. Zur Lösung gibt man 2,7 ml Methanol und anschließend versetzt man die Lösung mit 65 ml MtBE. Nach etwa 1 min beginnt die Rekristallisation des MtBE-Solvates. Der gesamte Prozeß verläuft bei Raumtemperatur. Die Suspension wird 1 h bei 5°C gerührt, dann filtriert und der Filterkuchen mit 30 ml kaltem MtBE gewaschen. Nach der Trocknung beträgt die Ausbeute 84 Gew.-% des Einsatzmaterials. Das erhaltene Produkt ist wiederum ein MTBE-Solvat mit 22,3 % MtBE.Der Reinigungseffekt bezüglich der Hauptverunreinigungen wird in folgender Tabelle sichtbar:

| | Einsatzmaterial | Rekristallisat |
|---|---|---|
| E-Oxim | 97,7 F% | 98,8 F% |
| Z-Oxim | 1,7 F% | 1,0 F% |
| Dioxim | 0,4 F% | 0,04 F% |

### Beispiel 3

30 g MtBE-Solvat des Oxims J867 (MtBE-Solvat mit 20 bis 24 % MtBE) bwerden in 600 ml Wasser suspendiert und auf 70°C aufgeheizt und 30 min suspendiert. Die Suspension wird filtriert und im Luftstrom trockengesaugt. Anschließend wird das Kristallisat im Vakuum 3h bei 70°C und < 5 mbar getrocknet.
Es werden 24 g hochkristallines Oxim J867 erhalten.
Die Phasenumwandlung in heißem Wasser als auch die Trocknung führen zu keiner signifikanten Zunahme des Z-lsomeren und des Aldehyds.

| | Z-Oxim (HPLC) | Aldehyd (HPLC) |
|---|---|---|
| MTBE-Solvat des Oxims J867 | 1,58 F% | 0,17 F% |
| Hochkristallines Ansolvat des Oxims J867 | 1,61 F% | 0,13 F% |

Im XRPD wurde hinsichtlich der Intensität und Lage der Reflexe und in der DSC hinsichtlich der Schmelzwärme eine Übereinstimmung mit den Daten der hochkristallinen Ansolvatform des Oxims J867 nachgewiesen.

## Patentansprüche

1. Amorphes 11β-[4E-(Hydroxyiminomethyl)phenyl]-17α-methoxymethyl-17β-methoxy-estra-4,9-dien-3-on (J 867), **dadurch gekennzeichnet, daß** keine kristallinen Reflexe im Röntgen-Pulverdiffraktogramm (XRPD) zu sehen sind (Figur 1a und 1b).

2. Amorphes 11β-[4E-(Hydroxyiminomethyl)phenyl]-17α-methoxymethyl-17β-methoxy-estra-4,9-dien-3-on, **dadurch gekennzeichnet, daß** die Modifikation durch das in Figur 2 dargestellte IR-Spektrum charakterisiert ist.

3. Hochkristallines 11β-[4E-(Hydroxyiminomethyl)phenyl]-17α-methoxymethyl-17β-methoxy-estra-4,9-dien-3-on-Ansolvat.

4. Hochkristallines 11β-[4E-(Hydroxyiminomethyl)phenyl]-17α-methoxymethyl-17β-methoxy-estra-4,9-dien-3-on-Ansolvat nach Anspruch 3, **dadurch gekennzeichnet, daß** die Kristallform durch das in Figur 3 dargestellte IR-Spektrum charakterisiert ist.

5. Hochkristallines 11β-[4E-(Hydroxyiminomethyl)phenyl]-17α-methoxymethyl-17β-methoxy-estra-4,9-dien-3-on-Ansolvat nach Anspruch 3, **dadurch gekennzeichnet, daß** die Kristallform durch das in Figur 1c (Tabelle 1) dargestellte Röntgen-Pulverdiffraktogramm charakterisiert ist.

6. Kristalline Solvate von 11β-[4E-(Hydroxyiminomethyl)phenyl]-17α-methoxymethyl-17β-methoxy-estra-4,9-dien-3-on, nämlich
das Methyl-tert.-butylether-Solvat, wobei die kristalline Form durch das in Figur 4c (Tabelle 2) dargestellte Röntgen-Pulverdiffraktogramm **gekennzeichnet** ist,
das Aceton-Solvat, wobei die kristalline Form durch das in Figur 4b (Tabelle 2) dargestellte Röntgen-Pulverdiffraktogramm **gekennzeichnet** ist, und
das Ethanol-Solvat, wobei die kristalline Form durch das in Figur 4a (Tabelle 2) dargestellte Röntgen-Pulverdiffraktogramm **gekennzeichnet** ist.

7. Verfahren zur Herstellung eines kristallinen Solvats von 11β-[4E-(Hydroxyiminomethyl)phenyl]-17α-methoxymethyl-17β-methoxy-estra-4,9-dien-3-on nach Anspruch 6, umfassend die folgenden Schritte:
a) Herstellung eines Solvats von 11β-[4E-(Hydroxyiminomethyl)phenyl]-17α-methoxymethyl-17β-methoxy-estra-4,9-dien-3-on und Auflösen des Solvats oberhalb der Sättigungslöslichkeit in einem Lösungsmittel, das unterschiedlich vom Solvat-Lösungsmittel ist und in welchem die Solvat-Kristallstruktur instabil ist,
b) gegebenenfalls Zugabe eines weiteren Lösungsmittels als Rekristallisations-Inhibitor und
c) Zugabe eines Solvatbildner-Lösungsmittels und Kristallisieren des Solvats.

8. Verfahren zur Herstellung von amorphem 11β-[4E-(Hydroxyiminomethyl)phenyl]-17α-methoxymethyl-17β-methoxy-estra-4,9-dien-3-on, umfassend die Trocknung des gemäß Anspruch 6 erhaltenen kristallinen Solvats von 11β-[4E-(Hydroxyiminomethyl)phenyl]-17β-methoxymethyl-estra-4,9-dien-3-on unterhalb des Glasübergangspunkts der amorphen Substanz, wobei die Trocknung des Solvats während der Desolvatisierungsphase mit einer Heizrate im Produkt von mindestens 0,5°C/min oder aus einer Lösung des Solvats durch Sprühtrocknung erfolgt.

9. Verfahren zur Herstellung des hochkristallinen 11β-[4E-(Hydroxyiminomethyl)phenyl]-17α-methoxymethyl-17β-methoxy-estra-4,9-dien-3-on-Ansolvats nach einem der Ansprüche 3 bis 5, **dadurch gekennzeichnet, daß** eine der in Anspruch 6 genannten Solvatformen bei einer Temperatur von 50 bis 100 °C in Wasser suspendiert wird.

10. Pharmazeutische Zusammensetzung, umfassend amorphes 11β-[4E-(Hydroxyiminomethyl)phenyl]-17α-methoxymethyl-17β-methoxy-estra-4,9-dien-3-on und/oder hochkristallines 11β-[4E-(Hydroxyiminomethyl)phenyl]-17α-methoxymethyl-17β-methoxy-estra-4,9-dien-3-on-Ansolvat nach einem der Ansprüche 1 bis 5, in Kombination mit einem pharmazeutisch verträglichen Träger und/oder Verdünnungsmittel.

11. Amorphes 11β-[4E-(Hydroxyiminomethyl)phenyl]-17α-methoxymethyl-17β-methoxy-estra-4,9-dien-3-on und/oder hochkristallines 11β-[4E-(Hydroxyiminomethyl)phenyl]-17α-methoxymethyl-17β-methoxy-estra-4,9-dien-3-on-Ansolvat gemäß einem der Ansprüche 1 bis 5 zur Anwendung als Arzneimittel.

12. Verwendung von amorphem 11β-[4E-(Hydroxyiminomethyl)phenyl]-17α-methoxymethyl-17β-methoxy-estra-4,9-dien-3-on und/oder hochkristallinem 11β-[4E-(Hydroxyiminomethyl)phenyl]-17α-methoxymethyl-17β-methoxy-estra-4,9-dien-3-on-Ansolvat nach einem der Ansprüche 1 bis 5 zur Herstellung eines Arzneimittels für die weibliche Fertilitätskontrolle, zur Behandlung von hormonabhängigen gynäkologischen Störungen und für die Hormon-Replacement-Therapie.
